Europäisches Patentamt

(19) European Patent Office  (11) Publication number: **0 081 962**
A2
Office européen des brevets

(12) # EUROPEAN PATENT APPLICATION

(21) Application number: **82306498.5**  (51) Int. Cl.³: **A 61 K 7/30**

(22) Date of filing: **07.12.82**

---

(30) Priority: **08.12.81 US 328570**

(71) Applicant: **WARNER-LAMBERT COMPANY, 201 Tabor Road, Morris Plains New Jersey 07950 (US)**

(43) Date of publication of application: **22.06.83 Bulletin 83/25**

(72) Inventor: **Mohrle, Raymond L., Jr., 11 Hill Road, Denville New Jersey 07834 (US)**

(84) Designated Contracting States: **BE CH DE FR GB IT LI SE**

(74) Representative: **Jones, Michael Raymond et al, Haseltine Lake & Co. 28 Southampton Buildings Chancery Lane, London WC2A 1AT (GB)**

---

(54) **Denture cleansing composition.**

㊗ A rapidly disintegrating tablet form of a stable effective and highly effervescent chlorineyielding denture cleansing composition reactive under aqueous conditions is obtained by utilizing a combination of an oxygen-yielding perborate with a hypochlorite-yielded chloroisocyanurate, when a substantial proportion of the total oxygenyielding perborate in the denture cleansing composition is present in an anhydrous form. The cleansing composition also includes alkaline agents which provide a pH of about 11 in the aqueous medium in which the tablets are normally dissolved.

EP 0 081 962 A2

-1-

DENTURE CLEANSING COMPOSITION

This invention relates to a denture cleansing composition.

Denture cleansers in the form of effervescent tablets which rely for their cleansing activity on a combination of both an alkali metal perborate as an oxygen source and an alkali metal dichloroisocyanurate as a hypochlorite chlorine source have been disclosed in the prior art. More particularly, such cleansing compositions are disclosed in both U.S. Patents Nos. 3,821,117 and 3,936,385. Considerable pertinent prior art is referred to in the latter patent. An alkaline reacting material is included with the above reactants so that the aqueous medium in which the cleansing composition is dissolved will have a pH which may be about pH 9 but is usually from about pH 11 to pH 11.5.

In the U.S. Patent No. 3,821,117 a more rapid dissolution in water of the cleanser tablets disclosed therein having these specific active ingredients is apparently obtained if from 4 to 20% by weight of benzoic acid or a benzoic acid compound is incorporated into the combination prior to being compressed into tablets. The particular inventive

0081962

concept described in U.S. Patent No. 3,936,385 lies in employing the combination of perborate or other peroxygen compound with the hypochlorite-yielding chlorine compound in such a stoichiometric ratio that the final alkaline cleansing solution, obtained on dissolution of the tablet and after release of free oxygen has ceased, will contain free hypochlorite chlorine.

Both of these prior art patents contain a broad disclosure of those alkaline agents, oxygen-yielding peroxygen compounds, and hypochlorite-yielding chlorine compounds which are sutiable for use in formulating the denture cleansing effervescent tablets described and claimed. The tabletting excipients and techniques employed in the preparation of those tablets, including the mixing steps and the relative amounts of active substances employed by percentages, are also given in considerable detail, and all of this disclosure is incorporated herein by reference as pertinent and applicable prior art. A careful review of the disclosure in U.S. Patent No. 3,936,385 demonstrates that the formulation of the compositions disclosed therein has been the subject of a considerable amount of research.

According to the present invention, there is provided a solid compressed denture cleansing composition which is highly effervescent when added to water, the composition comprising a mixture of an oxygen-yielding perborate with a hypochlorite-yielding chloroisocyanurate chlorine compound and including at least one alkaline salt capable of producing a pH of about 11 when dissolved in water, wherein a substantial proportion of the total perborate present in the composition is in the anhydrous form.

With the compositions of the present invention, rapid and highly satisfactory disintegration of the tablet together with voluminous effervescence is achieved by a substantial proportion of the perborate incorporated as an active ingredient in the tablet being present in anhydrous form. Thus, for example, when the tablet comprises the combination of an alkali metal perborate and an alkali metal dichloroisocyanurate as described above in the prior art, significantly improved and very rapid tablet disintegration and effervescence are achieved when at least 20% of the total alkali metal perborate present in the tablet is in the anhydrous form.

As described, the preferred compound which is employed as the hypochlorite-yielding chlorine compound in these compositions is an alkali metal chlorooisocyanurate such as sodium dichloroisocyanurate or potassium dichloroisocyanurate, or it may be trichlorocyanuric acid or a complex salt of two or more of these compounds.

The oxygen-yielding perborate employed in these effervescent denture cleansing compositions is preferably an alkali metal perborate. Sodium perborate is preferred owing to its ready commercial availability and its reasonable cost. In formulating the novel denture cleansing compositions of the present invention, for example, a mixture of sodium perborate monohydrate containing a substantial proportion of anhydrous sodium perborate is preferably employed. At least 20% of the total sodium perborate employed should be in the form of anhydrous sodium perborate. Anhydrous sodium perborate is readily available commercially and can be obtained by heating the corresponding sodium perborate monohydrate or sodium perborate tetrahydrate.

The desired degree of alkalinity required to produce denture cleansing compositions which are satisfactorily effervescent from a mixture of sodium perborate and sodium or potassium dichloroisocyanurate, as described, is obtained by incorporating an alkaline agent such as tetrapotassium pyrophosphate or trisodium phosphate into the mixture. In addition to containing one, or both, of the above alkaline agents the denture cleanser composition can also contain, in addition, a highly alkaline agent such as sodium metasilicate. When dissolved in an aqueous medium these alkaline agents yield aqueous solutions which are at a pH of 11 or more. This degree of alkalinity is ample to ensure active effervescence of the free oxygen formed as well as the production of free hypochlorite chlorine.

The mixture which is compressed into tablets to form these improved effervescent denture cleaning compositions may also include a surface active agent such as sodium, potassium or calcium alkyl aryl sulphonate, for example, to impart foaming and detergent properties to the cleansing solution obtained on dissolution of the tablet.

In order further to illustrate the present invention the following Examples are given.

### EXAMPLE 1

The following ingredients were thoroughly mixed in the proportions given below and then formed into tablets by conventional tabletting techniques.

| Ingredients | Grams Per 1000 Tablets |
|---|---|
| Sodium Perborate, Anhydrous | 800.0 |
| Sodium Perborate, Monohydrate | 200.0 |
| Tetrapotassium Pyrophosphate, Powder | 300.0 |
| Sodium Metasilicate, Anhydrous | 1200.0 |
| Aryl Alkyl Sulphonate (Calsoft F-90) | 30.0 |

| | |
|---|---|
| Sodium Benzoate, Fine Powder (Tabletting lubricant) | 50.0 |
| Potassium Dichloroisocyanurate | 1000.0 |
| | 3580.0 |

The tablets obtained dissolved thoroughly in water at room temperature in less than 2 minutes. When one tablet was dissolved in 120 ml of water at 45°C the resulting solution had a pH of 11 and, because of the relatively high ratio of potassium dichloroisocyanurate present, contained an excess of hypochlorite.

EXAMPLE 2

The following ingredients were thoroughly mixed in the proportions given below and then tabletted by conventional tabletting procedures.

| Ingredients | Grams Per 1000 Tablets |
|---|---|
| Sodium Perborate, Anhydrous | 640.0 |
| Sodium Perborate, Monohydrate | 600.0 |
| Tetrapotassium Pyrophosphate, Powder | 400.0 |
| Sodium Metasilicate, Anhydrous | 600.0 |
| Aryl Alkyl Sulphonate (Calsoft F-90) | 30.0 |
| Sodium Benzoate, Fine Powder (Tabletting lubricant) | 48.0 |
| Potassium Dichloroisocyanurate) | 100.0 |
| | 2418.0 |

When dissolved in water at room temperature, complete solution was achieved in less than 2 minutes. A solution pH of 11.2 was obtained when one tablet was dissolved in 120 ml of water at 45°C. No chlorine odour was detected.

CLAIMS:

1. A solid compressed denture cleansing composition which is highly effervescent when added to water, the composition comprising a mixture of an oxygen-yielding perborate with a hypochlorite-yielding chloroisocyanurate chlorine compound and including at least one alkaline salt capable of producing a pH of about 11 when dissolved in water, wherein a substantial proportion of the total perborate present in the composition is in the anhydrous form.

2. A composition in accordance with Claim 1, wherein the anhydrous perborate constitutes at least 20% of the total perborate present.

3. A composition in accordance with Claim 1 or 2, wherein the perborate is an alkali perborate and the hypochlorite-yielding chlorine compound is an alkali metal dichloroisocyanurate.

4. A composition in accordance with Claim 3, wherein the perborate is sodium perborate and the hypochlorite-yielding chlorine compound is potassium dichloroisocyanurate.